# EUROPEAN PATENT APPLICATION

(11) **EP 3 842 038 A1**
(43) Date of publication of application: **30.06.2021**
(21) Application number: 20217089.0
(22) Date of filing: 23.12.2020
(51) Int. Cl.: A61K 9/50, A61K 9/16, A61K 31/295, A61K 31/198, A61K 33/26

(54) **MICROENCAPSULATED FORMULATION COMPRISING IRON AND CYSTEINE**

(30) Priority: 23.12.2019 IT 201900025393
(71) Applicant: Pharma Line S.r.l., 20154 Milano (IT)
(72) Inventor: SIDOTI, Rossana, 20145 Milan (IT); MANONI, Aristide, 20154 Milan (IT)
(74) Representative: PGA S.p.A.

(57) **Abstract**

The present invention relates to a formulation microencapsulated with a coating matrix, wherein said formulation comprises or, alternatively, consists of: (a) iron and (b) cystine or a derivative thereof. Furthermore, the present invention relates to compositions comprising said microencapsulated formulation and, optionally, (c) at least one vitamin B, (d) vitamin C and/or (e) vitamin E. Lastly, the present invention relates to the use of said microencapsulated formulations and compositions thereof for the treatment of anaemias or iron deficiency, and of diseases, disorders or symptoms related thereto.

## Description

The present invention relates to a microencapsulated formulation with a coating matrix comprising at least one polymer, preferably at least one alginate or at least one lipid, wherein said formulation comprises or, alternatively, consists of: (a) iron and (b) cystine or a salt or derivative thereof. Furthermore, the present invention relates to compositions comprising said microencapsulated formulation and, optionally, (c) at least one vitamin B, (d) vitamin C and/or (e) vitamin E. Lastly, the present invention relates to the use of said microencapsulated formulations and compositions thereof for the treatment of anaemia or iron deficiency, and of diseases, disorders or symptoms related thereto.
Anaemia is a public-health problem that affects the people in rich and poor countries. Although the main cause of anaemia is iron deficiency, said cause is rarely present alone. More frequently, it co-exists with a number of other causes, such as malaria, parasitic infection, nutritional deficiency and hemoglobinopathies. Anaemia occurs at all stages of the life cycle, but is more common in women, pregnant women, children and the elderly. A 2008 WHO analysis reported that anaemia affected 24.8% of the world population, including 42% of pregnant women, 30% of non-pregnant women and 47% of preschool children. Estimates in 2011 suggest that anaemia affects about 800 million children and women. When anaemia is associated with another disease, such as cancer or kidney disease, it has adverse effects on the underlying disease, including worsening symptoms, faster progression of the disease and worse prognosis. Anaemia is a prevalent condition that is not recognised and treated properly. However, besides entailing considerable costs for patients and national health services, it is also a burden on the health and the quality of life of the diagnosed people. Reducing anaemia is recognised as an important component of women's and children's health and the second global nutritional goal for the 2025 goals for a 50% reduction in anaemia in women of reproductive age.

Furthermore, iron deficiency and oxidative stress are a vicious circle, given that, especially in the liver and red blood cells, 4 iron atoms are required to neutralise reactive oxygen species (ROS). In addition, low-grade systemic inflammation conditions cause oxidative stress that damages all cells, including those most markedly involved in iron metabolism and in the preservation of iron homeostasis. Oxidative stress is involved in the increased eryptosis that characterises the forms of anaemia that affect people with low-grade systemic inflammation. In particular, the liver is very sensitive to oxidative stress and the liver is responsible for iron and copper deposition and for the synthesis of proteins involved in iron metabolism (transferrin, ferritin, ceruloplasmin, etc.). The impairment of hepatic functions induced by oxidative stress causes an altered management of iron metabolism.

Lastly, iron is essential for the proper functioning of mitochondria, given that it is necessarily included in cytochromes, and therefore for an adequate supply of energy required for the performance of all the functions of the organism, comprised the synthesis of endogenous substances with antioxidant function. Anaemia is a common syndrome in elderly subjects. Various studies have revealed a relation between anaemia, bone fragility and age-related chronic inflammation, based on immunosenescence. Inflammation is characterised by age-related chronic upregulation of the inflammatory immune response with increased levels of proinflammatory cytokines, such as interleukin 1 (IL 1), IL-6 and tumour necrosis factor (TNF). Inflammation is believed to be a consequence of permanent exposure to the antigenic load and environmental free radicals, with ensuing in chronic proinflammatory condition.

Erythrocyte apoptosis (or eryptosis or red blood cell programmed death) is a physiological mechanism for removing defective erythrocytes from blood circulation. Factors triggering an excessive eryptosis include oxidative stress and multimorbidity. Eryptosis has been described in relation to various morbidities such as diabetes mellitus, chronic heart disease, chronic renal failure and dehydration. These comorbidities are associated with chronic inflammation and ROS and they could therefore be considered responsible for increased eryptosis. Although the exact underlying mechanisms are still unclear, a close link between low-grade systemic inflammation and anaemia, in which ROS play a key role, can be assumed.

The drugs or supplements used to date for the treatment of anaemia and/or iron deficiency conditions and diseases, disorders or symptoms related thereto are not always effective, or, they lead to only partial or only temporary resolution of the disorder. Furthermore, the products used to date for the supplementation of an iron deficiency are often poorly tolerated at the gastrointestinal level and they have a low percentage of intestinal absorption and blood bioavailability of iron by the body, particularly when the product is administered through the oral route.

The type of treatment of anaemia depends on the cause and severity of the disease. In particular, besides iron, an oral product for anaemia may include vitamin and mineral supplements in case of specific deficiencies, vitamin B12 (by injection) for pernicious anaemia, antibiotics if caused by infection or anti-inflammatory agents. If anaemia is characterised by high iron deficiency, the treatment may provide for iron injections or blood transfusion or oxygen therapy. The guidelines indicate iron formulations for oral use as the first-line strategy for the treatment of sideropenic anaemia, except for particularly severe conditions in which intravenous therapy or blood transfusion are to be used.

The products most commonly used for oral administration are products based on iron sulphate. However, the main disadvantage of this treatment lies in the poor tolerability that often forces patients to abandon therapy. This is particularly common in the elderly subjects or in subjects with gastrointestinal disorders. Furthermore, the common deficiency of vitamins needed to support recovery from erythropoiesis often requires the use of supplements containing vitamins, for example vitamins of group B.

Therefore, there is a high need to provide innovative formulations or compositions based on iron that are highly effective in the preventive and/or curative treatment of anaemia and/or iron deficiency conditions and diseases, disorders or symptoms related thereto (for example, tiredness and sensation of fatigue), or, alternatively, highly effective for non-therapeutic supplementation of iron in non-pathological subjects (for example sportsmen/sportswomen).

Furthermore, the need is felt to provide innovative formulations or compositions based on iron for an effective preventive and/or curative treatment of said iron deficiency capable of allowing a rapid and almost total restoration of the normal iron values (standard values for a healthy subject), that can be used by all classes of subjects (such as sportsmen/sportswomen, pregnant or breastfeeding women, the elderly and paediatric subjects), well tolerated and devoid of significant side effects.

Lastly, the need is felt to provide compositions or formulations for an effective preventive and/or curative treatment of the aforementioned diseases that are easy to prepare and cost-effective.

Following extensive research and development activity, the Applicant addresses and solves the aforementioned needs by providing a microencapsulated formulation with a coating matrix A comprising at least one polymer, preferably at least one alginate salt or at least one lipid, wherein said formulation comprises or, alternatively, consists of: (a) iron, in the form of an iron (III) or iron (II) cation, and (b) cystine or a salt or derivative thereof (in short, microencapsulated formulation of the invention), and compositions comprising said microencapsulated formulation and, optionally, (c) at least one vitamin B and/or (d) vitamin C and/or (e) vitamin E and/or (f) folic acid (in short, compositions of the invention).

The formulations comprising (a) and (b) microencapsulated with polymers, preferably alginates or lipids, and the compositions thereof subject of the present invention are highly effective in the curative and/or preventive treatment of anaemia and/or iron deficiency and improved with respect to the treatments with products currently available in the prior art.

In particular, (b) cystine or a salt or derivative thereof, wherein said derivative is preferably selected from cysteine, acetylcysteine and salts thereof, prevents the increase of eryptosis from reducing the average life of red blood cells. As a matter of fact, cystine or a salt or derivative thereof present in the microencapsulated formulation of the invention is a source of cysteine (each cystine molecule provides two cysteine molecules). Cysteine is the limiting precursor in glutathione synthesis, and glutathione is the main endogenous antioxidant that combats the oxidative stress responsible for the increase in eryptosis (i.e. the decrease in the average life of red blood cells).
Furthermore, glutathione has important functions in iron metabolism and cellular iron homeostasis through the formation of various iron complexes, such as iron (II) glutathione, glutathionyl-dinitrosyl-iron complexes, or the coordination of iron-sulfur (FeS) cluster by glutaredoxins.
As a result, in order to make the treatment of anaemia effective, it is essential to increase glutathione levels at the same time as administration of iron, in order not only to reduce the degree of eryptosis, but above all to ensure adequate cellular iron homeostasis and iron metabolism.

In summary, the role of cystine or a derivative thereof in the formulation of the present invention can be identified as follows:
- given the many functions thereof, glutathione is fundamental to combat oxidative stress and to preserve the homeostasis of the whole body, in particular iron homeostasis and the integrity and efficiency of erythrocytes;
- glutathione synthesis depends both on the adequate supply of cysteine, and on the correct functioning of the methionine and folate cycle and of the reactions of the transsulfuration pathway;
- cysteine is unstable and spontaneously gives rise to cystine in an autoxidation reaction which generates potentially harmful free radicals; for this reason, the administration of cystine, which is readily absorbed by the intestine, is preferred to administration of cysteine;
- a suitable supply of cysteine (preferably in the form of cystine) and complex B vitamins, allows the development of the reactions which are involved both in glutathione synthesis starting from the cysteine, and in the transsulfuration pathway.
Without prejudice to the fact that for the purpose of the effectiveness thereof the formulation of the present invention may comprise cystine or a derivative thereof, such as cysteine or N-acetylcysteine, according to an aspect of the invention cystine is preferable with respect to both cysteine and N-acetylcysteine given that: cysteine is more bioavailable with respect to N-acetylcysteine, whereas cystine is preferable with respect to cysteine because it does not undergo the oxidation process characteristic of cysteine, which generates free radicals with oxidizing effect harmful to tissues.

The presence of a coating matrix in the formulation of the invention which microencapsulates both the iron cation (a) and cystine or a salt or derivative thereof (b) ensures their high bioavailability, avoiding the attack of gastric acids and enhancing the absorption thereof at duodenal level. Furthermore, microencapsulation ensures a high digestibility of the formulation of the invention and of the compositions thereof, while avoiding both the metallic aftertaste and the classical gastrointestinal disorders related with administration of iron through the oral route.

Furthermore, said coating matrix in the formulation of the invention which microencapsulates both the iron cation (a) and cystine or a salt or derivative thereof (b) may allow modulated release of the individual active components (a) and (b) independently with respect to each other, such as delayed release and/or a prolonged release.

The presence of the iron (III) or (II) cation in the microencapsulated formulation of the invention has the effect of supporting haemoglobin synthesis, ensuring high efficacy of the formulation and of the compositions thereof subject of the present invention which are highly tolerated thanks to microencapsulation. In particular, microencapsulation maximises the physiological absorption of iron through the physiological pathway of duodenal enterocytes, with respect to iron sulphate, considered the reference standard for iron therapy of sideropenic anaemia through the oral route. Furthermore, given that iron (III) cation is microencapsulated, the formulations and compositions of the present invention do not exhibit the same severe side effects as iron sulphate, which force one in three patients to discontinue therapy prematurely.

In addition, besides iron and cystine or a salt or derivative thereof, the presence of vitamins B - in particular B6 and B12 - in the compositions of the present invention enhances the normal formation of red cells and therefore enhancing the treatment of tiredness and fatigue.

Lastly, besides iron and cystine or a salt or derivative thereof, the presence of Vitamin C in the compositions of the present invention enhances the absorption of iron by the body of the subject to whom the composition of the invention is administered. Vitamin C has a strong reducing power and it plays a significant role in the absorption of iron at intestinal level; furthermore, at systemic level it essential to allow transferrin to release iron to cells and to protect tissues from oxidative stress.

Vitamins B and/or Vitamin C, when present in the composition of the invention together with the microencapsulated iron and cystine formulation, support the recovery of erythropoiesis induced by the increased availability of iron and they support cellular replication of erythrocytes stimulated by the compensation of the sideropenia condition.

Vitamin B2 is indispensable for the activation of folate (vitamin B9), vitamin B6 and vitamin B12. Furthermore, it is indispensable for the synthesis and metabolism of vitamin D. Vitamin D is involved in erythropoiesis, decrease in proinflammatory cytokines and decrease in hepcidin mRNA transcription. Vitamin B6 is involved in iron metabolism and it acts synergistically with vitamin B12 and folate (vitamin B9).
Vitamin B12 deficiency determines megaloblastic anaemia (pernicious anaemia).

Vitamin E protects the double phospholipid membrane layers and proteins inserted into cell membranes against radical oxidizing species and it acts synergistically with vitamin C. Vitamin E deficiency may determine oxidative stress conditions that make the erythropoiesis processes ineffective and enhance increase in the lysis of erythrocytes.

Therefore, an object of the present invention is to provide an innovative formulation of the microencapsulated active components, such as (a) iron and (b) cystine or a salt or derivative thereof, and compositions thereof optionally comprising (c), (d), (e) and/or (f), preferably for oral use, which are capable of:
- providing high bioavailability of iron and cystine or derivatives thereof,
- combatting oxidative stress,
- combatting eryptosis and supporting the recovery of erythropoiesis,
- modulating iron homeostasis,
- reducing inflammation,
- modulating the release of active components (e.g. delayed release, prolonged release) and the bioavailability thereof,
- masking the taste and/or odour of iron,
- improving the shelf life of the formulations or compositions of the invention.

The microencapsulated formulation and the compositions thereof subject of the present invention are effective in the treatment of anaemia or iron deficiency conditions, preferably, in a condition of:
- food deficiency (e.g. unbalanced diet) or increased dispersion (e.g. chronic kidney disease) or increased demand (e.g. chronic diseases of various origin) of iron, amino acids and vitamins;
- low-grade chronic inflammation (low-grade systemic inflammation) and oxidative stress.

Low-grade systemic inflammation and oxidative stress play a role in the pathogenesis of anaemia. Therefore, with the microencapsulated formulation and the compositions thereof subject of the present invention the Applicant addresses the nutritional deficiency involved in iron metabolism and while combatting oxidative stress at the same time.

Microencapsulation with a coating matrix based on polymers, preferably alginates or lipids, makes the oral administration of said active components (a) iron and (b) cystine (or derivatives thereof) highly effective following a gastroprotection thereof from the acidic environment and/or an increase in the ability thereof to pass through the intestinal barrier (increased intestinal permeability), with ensuing increase in the gastrointestinal absorption of said active components and, therefore, in the blood bioavailability thereof, and with decrease in subjective variability to the effectiveness of the composition comprising the microencapsulated formulation subject the present invention.

In the context of the present invention, the terms gastrointestinal absorption and intestinal absorption are used interchangeably.

Furthermore, said high efficacy of the microencapsulated formulations and compositions thereof subject of the present invention and/or said increased blood bioavailability of the active components of said microencapsulated formulations results in achieving a greater effect considering the same daily intake administered to a subject in need or the use of a lower daily intake to achieve the same effect, with respect to the products of the prior art.

Said high efficacy of the microencapsulated compositions and formulations subject of the present invention and/or said increased blood bioavailability of the active components thereof also results in a decrease in the onset of said microencapsulated compositions and formulations after administration to a subject in need, with respect to the products of the prior art.
In the context of the present invention, the term "*onset*" is used to indicate the latency time between the administration of a composition/product and the pharmacological effect thereof. In other words, onset is the time that elapses from when the composition/product is administered up to the partial or total pharmacological effect.

In addition, the high efficacy of the microencapsulated compositions and formulations subject of the present invention are due to the selected and specific combination of the active components present in the microencapsulated compositions or formulations and/or to the synergy of said active components, such as (a) iron, (b) cystine or a derivative thereof and, if present, (c) at least one vitamin B, preferably vitamin B2, B6, B9 and/or B12, (d) vitamin C and/or (e) vitamin E and/or (f) folic acid.

Furthermore, the microencapsulated compositions or formulations subject of the present invention are stable over time from the chemical-physical, shelf-life and organoleptic point of view; advantageously, they are more stable than the products of the prior art.

Furthermore, the microencapsulated compositions and formulations of the present invention do not have significant side effects, they are well tolerated and, therefore, can be administered to all subjects, in particular also to pregnant or breastfeeding female subjects, subjects in paediatric age, sportsmen/sportswomen and the elderly.

Lastly, the microencapsulated compositions and formulations of the present invention are easy to prepare and cost-effective.

These and other objects which will be apparent from the detailed description that follows are achieved by the microencapsulated formulations and by the compositions of the present invention thanks to the technical characteristics reported in the present description and claimed in the attached claims.

### FIGURES

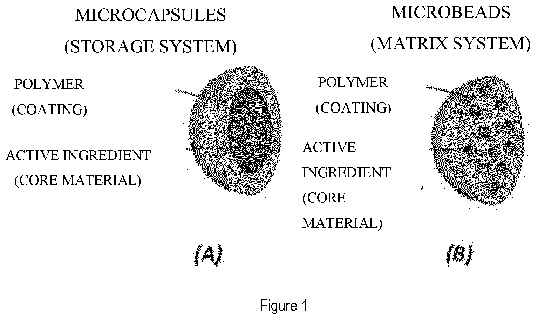

Figure 1: graphical representation of microcapsule structures of the microencapsulated formulation comprising (a) and (b) subject of the present invention.

### SUMMARY OF THE INVENTION

In a first aspect, the present invention relates to a microencapsulated formulation with a coating matrix, wherein said formulation comprises (a) iron and (b) cystine or a derivative thereof, such as microencapsulated cysteine or N-acetylcysteine, and wherein said coating matrix comprises at least one alginate salt or at least one phospholipid or at least one polymer other than an alginate salt.

In a second aspect, the present invention relates to a composition comprising said microencapsulated formulation.

In a third aspect, the present invention relates to said microencapsulated formulations and compositions thereof for use as medicament, such as for use in a method for the preventive or curative treatment of anaemia or an iron deficiency, and diseases or symptoms related thereto.

In a fourth aspect, the present invention relates to a method for the preventive or curative treatment of anaemia or an iron deficiency, and diseases or symptoms related thereto, wherein said method provides for the administration of said microencapsulated formulation or compositions thereof in a therapeutically effective amount to subjects in need.

In a fifth aspect, the present invention relates to the non-therapeutic use of said microencapsulated formulations and compositions thereof in healthy subjects, for example sportsmen/sportswomen, for an increase in physical capacities and physical performance.

In a sixth aspect, the present invention relates to a process for preparing said microencapsulated formulation (microencapsulation method).

In a seventh aspect, the present invention relates to a microencapsulated formulation obtained using the microencapsulation method of the invention.

### DETAILED DESCRIPTION OF THE INVENTION

Forming an object of the present invention is a microencapsulated formulation (in short, formulation of the invention or microencapsulated formulation of the invention) with a coating matrix A, wherein said formulation comprises or, alternatively, consists of:
(a) iron, wherein said iron is an iron (III) or iron (II) cation, and
(b) cystine or a salt or derivative thereof; and
wherein said coating matrix A comprises or, alternatively, consists of at least one polymer, preferably at least one alginate salt or at least one lipid; and, optionally, at least one acceptable pharmaceutical or food grade additive and/or excipient.

In the context of the present invention, with reference to the formulation subject of the invention, the terms "microencapsulated" or "coated" or "covered" or "embedded" are synonyms that can be used interchangeably. For the sake of brevity, in the context of the present invention only the term "microencapsulated formulation" is mentioned.

Preferably, the microencapsulated formulation subject the present invention is in solid state with a core/shell A structure A or a matrix B structure or a combined A+B structure, as described hereinafter in the present invention.

Cystine (IUPAC Name (2R,7R)-3,3'-dithiobis(2-aminopropanoic acid, common name L-(-)-cystine or (2S,7S)-(-)-cystines or dicysteine) is a sulfated amino acid characterised by C₆H₁₂N₂O₄S₂ obtained by means of oxidative reaction of two cysteine molecules for example (CAS No. 56-89-3). An example of cystine that can be used in the context of the present invention is cystine having the technical characteristics of the commercial product Cistina-L Base manufactured by Polichimica (Italy), as described in the Examples.

In the context of the present invention, the term (b) is used to indicate "cystine or a salt or derivative thereof, preferably "cystine or a salt thereof".
In the context of the present invention, with the term "derivative" referred to cystine it is used to indicate any cystine derivative suitable to provide cystine or cysteine, preferably said cystine derivative is selected from cysteine or acetylcysteine or salts thereof.
In the context of the present invention, the term (a) is understood to mean "(a.i) or (a.ii)", wherein (a.i) and (a.ii) have the technical characteristics according to any one of the present embodiments.
In the context of the present invention, the terms "acetylcysteine" and "N-acetylcysteine" are synonyms and used interchangeably.

According to a preferred embodiment of the microencapsulated formulation comprising (a) and (b) subject of the present invention, said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate. Preferably, said alginate present in the coating matrix A has an average molecular weight comprised in the range from 10000 to 600000 (for example, 50000, 100000, 150000, 200000, 250000, 300000, 350000, 400000, 450000, 500000, or 550000), preferably from 100000 to 400000, more preferably from 150000 to 300000.

Sodium alginate is a chemical compound formed from the sodium salt of alginic acid, with minimum formula NaC₆H₇O₆. Advantageously, sodium alginate is extracted from the cell walls of the algae, it has the appearance of a gum. The sodium alginate generally used in the food or pharmaceutical industry is an alginate E401 (E401 means: food additive permitted by European legislation and regulated by the Italian Ministerial Decree No. D.M.1996.)
Alginic acid, also referred to as algin, is a polysaccharide widely present in the cell walls of brown algae (for example, brute or molecular formula (C₆H₈O₆)n, Molecular mass (u) from 10000 to 600000).

An example of sodium alginate that can be used in the context of the present invention is a sodium alginate originating from alga having CAS No. 9005-38-3, purified in stearic acid originating from palm oil having CAS No. 57-11-4, for example a commercial product named NS ENTERIC® (Additive For Nutritional Enteric Coating 29z19241 Clear), as described in the Examples.

According to an alternative embodiment of the microencapsulated formulation subject of the present invention, comprising (a), such as (a.i) or (a.ii), and (b) cysteine or a salt or derivative thereof, said coating matrix A comprises or, alternatively, consists of at least one lipid selected from:
1) a phospholipid, more preferably a phosphoglyceride, even more preferably a phosphoglyceride selected from 1,2-diacyl-phospholipid, 1-alkyl-2-acyl-phospholopids, 1-alkenyl-2-acyl-phospholopids; even more preferably a diacyl-phospholipids selected from the group comprising or, alternatively, consisting of: phosphatidylcholine o lecithin (E322), phosphatidylserine, phosphatidylethanolamine, phosphatidylinositol, phosphatic acid and mixtures thereof; of said group preferably phosphatidylcholine or lecithin (E322), advantageously allergen free and or powdered or granular; more preferably a sunflower, corn or soy lecithin (E322); and
2) a lipid of plant origin, selected from the group comprising or, alternatively, consisting of:
   - glycerols or mono-alcohols or di-alcohols, mono-, di- or tri- esterified with saturated or unsaturated fatty acids (e.g. monounsaturated);
   - saturated or unsaturated free fatty acids (e.g. monounsaturated);
   - sucrose fatty acid esters (sucresters), preferably mixtures of mono- di- or tri- sucrose fatty acid esters;
      wherein said saturated or unsaturated fatty acids both free and esterified with glycerol or mono-alcohols or di-alcohols or sucrose, have a number of carbon atoms comprised in the range from C12 to C28, more preferably from C14 to C24, for example C16, C18, C20 and/or C22.

According to a further alternative embodiment of the microencapsulated formulation subject of the present invention, comprising (a), such as (a.i) or (a.ii), and (b) cysteine or a salt or derivative thereof, said coating matrix A comprises or, alternatively, it consists of at least one polymer other than an alginate, wherein said polymer has the technical characteristics suitable for microencapsulating said (a) and (b) and for ensuring, after oral administration of the microencapsulated formulation of the invention, a high degree of gastrointestinal absorption and bioavailability of (a) and/or (b), and/or a modulated release of (a) and/or (b), and/or a high degree of digestibility, and/or masking of the odour or taste of (a) and/or (b). Examples of polymers that can be used in the context of the present invention are ethyl cellulose and polyvinyl alcohol.

According to an embodiment of the microencapsulated formulation comprising (a) and (b) subject of the present invention, said active component (a) iron, wherein said iron is an iron (III) or iron (II) cation, may be (a.i) an iron salt or complex comprising an iron (III) or iron (II) cation (in short "(a.i)" or "(a.i) iron salt or complex") or, alternatively, it may be (a.ii) a microencapsulated iron comprising an iron (III) or iron (II) cation and a coating matrix B comprising at least one alginate salt which microencapsulates said iron (III) or iron (II) cation (in short "(a.ii)" or "(a.ii) iron microencapsulated with coating matrix B").

Advantageously, said (a.i) iron salt or complex (comprised in the formulation of the invention together with (b) cystine or salt or derivative thereof) is a water-soluble iron salt or complex.

As used in the context of the present invention, the term "water-soluble iron salt or complex refers to any iron salt freely soluble in water, such as iron pyrophosphate or ferric diphosphate, ferrous sulphate, ferrous gluconate, ferrous lactate and ferric ammonium citrate, as well as to any iron salt which is slowly soluble in water but easily soluble in dilute acids, such as ferrous fumarate, ferrous succinate and ferric saccharate.

According to an embodiment of the present invention, said microencapsulated formulation comprises or, alternatively, consists of:
- said (a) iron, wherein said iron is an (a.i) iron salt or complex comprising an iron (III) or iron (II) cation, wherein said iron salt or complex is selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
- said (b) cystine or a salt thereof, preferably crystalline cystine;
   and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate.

For example, said microencapsulated formulation comprises or, alternatively, consists of: ferric saccharate and (b) cystine or a salt thereof, preferably crystalline cystine, wherein said coating matrix A comprises or, alternatively, consists of at least one alginate salt, preferably sodium alginate.

According to an embodiment of the present invention, said microencapsulated formulation comprises or, alternatively, consists of:
- said (a) iron, wherein said iron is an (a.i) iron salt or complex comprising an iron (III) or iron (II) cation, wherein said iron salt or complex is selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
- said (b) cysteine or a salt thereof;
   and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate.

For example, said microencapsulated formulation comprises or, alternatively, consists of: ferric saccharate and (b) cysteine or a salt thereof, wherein said coating matrix A comprises or, alternatively, consists of at least one alginate salt, preferably sodium alginate.

According to an embodiment of the present invention, said microencapsulated formulation comprises or, alternatively, consists of:
- said (a) iron, wherein said iron is an (a.i) iron salt or complex comprising an iron (III) or iron (II) cation, wherein said iron salt or complex is selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
- said (b) acetylcysteine or a salt thereof;
   and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate.

For example, said microencapsulated formulation comprises or, alternatively, consists of: ferric saccharate and (b) acetylcysteine or a salt thereof, wherein said coating matrix A comprises or, alternatively, consists of at least one alginate salt, preferably sodium alginate.

According to an alternative embodiment of the present invention, said microencapsulated formulation comprises or, alternatively, consists of:
- said (a) iron, wherein said iron is an (a.ii) iron microencapsulated with the coating matrix B; and
- said (b) cystine or a salt or derivative thereof preferably crystalline cystine;
   and wherein said coating matrix A is selected from: at least one salt of an alginate (preferably sodium alginate), at least one lipid (according to any one of the embodiments described in the present invention, preferably comprising a phospholipid), and at least one polymer other than an alginate.

In an embodiment of the microencapsulated formulation of the invention (a) and (b), in said (a.ii) iron microencapsulated with the coating matrix B, the coating matrix B comprises at least one alginate salt selected from the group comprising or, alternatively, consisting of: sodium alginate, potassium alginate, magnesium alginate, ammonium alginate, calcium alginate and mixtures thereof; preferably calcium alginate. Preferably, said alginate present in the coating matrix A has an average molecular weight comprised in the range from 10000 to 600000 (for example, 50000, 100000, 150000, 200000, 250000, 300000, 350000, 400000, 450000, 500000, or 550000), preferably from 100000 to 400000, more preferably from 150000 to 300000.

Alternatively, said coating matrix B of said (a.ii) microencapsulated iron comprises or, alternatively, consists of at least one lipid or at least one polymer other than an alginate, according to the embodiments described for said lipid and said polymer with reference to the coating matrix A.

In an embodiment of the microencapsulated formulation of the invention comprising (a) and (b), said (a.ii) iron microencapsulated with the coating matrix B comprising an alginate, preferably calcium alginate, comprises the iron (III) or iron (II) cation in a total weight/weight percentage of (a.ii) microencapsulated iron (in short, % by w/w) comprised in the range from 25% to 55% (with respect to the total weight of (a.ii)), preferably from 30% to 50%, more preferably from 35% to 45% (for example about 40%).

In a further embodiment of the microencapsulated formulation of the invention comprising (a) and (b), said (a.ii) iron microencapsulated with the coating matrix B comprising an alginate, preferably calcium alginate, and comprising Fe(III) or Fe(II) in a % by w/w from 25% to 55% or from 30% to 50% or from 35% to 45%, further comprises a chelating agent, preferably selected from the group comprising or, alternatively, consisting of: tartaric, malic, succinic, fumaric, citric, lactic and oxalic acid, or a salt thereof, EDTA and sucrose. More preferably, the chelating agent is sucrose.
Advantageously, when the source of Fe(III) cation in the process for preparing (a.ii) is ferric saccharate (or ferric hydroxide sucrose complex, Fe(OH)O sucrose, CAS No. for example 8047-67-4), the sucrose of the ferric saccharate acts as a chelating agent.

It is clear that in order to achieve the objects of the present invention, as source of Fe(III) or Fe(ll) cation in the process for preparing (a.ii) there can be used any iron (III) or iron (II) salt or complex, preferably selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate.

Advantageously, said (a.ii) iron microencapsulated with the coating matrix B, comprised in the microencapsulated formulation of the invention together with (b), can be obtained by means of a microencapsulation process (for example spray method, mixing method, fluid bed method and others) and equipment known to the man skilled in the art.

Furthermore, the microencapsulation process for the preparation of (a.ii) iron microencapsulated with the coating matrix B and the microencapsulation process for the preparation of the microencapsulated formulation of the present invention comprising (a) iron, such as (a.i) or (a.ii), and (b) cystine or a salt or derivative thereof, can be two processes different from each other, for example the first a mixing process and the second a spray process, or the first a mixing or spray process and the second a fluid bed process. Alternatively, said two microencapsulation processes can be carried out by means of the same method and equipment, for example by means of a fluid-bed process.

Preferably, said (a.ii) iron microencapsulated with the coating matrix B comprised in the formulation of the invention together with (b), has a core/shell structure (structure A according to figure 1) wherein the core comprises the Fe(III) or Fe(ll) cation (preferably ferric saccharate) and the shell comprises the alginate salt (preferably calcium alginate).
An example of said (a.ii) iron microencapsulated with the coating matrix B, that can be used in the context of the present invention, is the commercial product "AB-Fortis®" (registered trademark, AB-Biotics, Spain) having the technical characteristics as reported in the Examples.

According to a preferred embodiment of the present invention, said microencapsulated formulation comprises or, alternatively, consists of:
- said (a) iron, wherein said iron is (a,ii) microencapsulated iron comprising an iron (III) or iron (II) cation and a coating matrix B comprising at least one alginate salt, preferably calcium alginate, which microencapsulates said iron (III) or iron (II) cation;
   preferably wherein said iron (III) or iron (II) cation derives from an iron salt or complex selected from: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
   preferably wherein said (a.ii) comprises Fe (III) or (II) in a % by w/w comprised in the range from 25% to 55% or from 30% to 50% or from 35% to 45% (e.g. about 40%); and
- said (b) cystine or a salt thereof or a derivative thereof (for example cysteine or acetylcysteine), preferably crystalline cystine;
   and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate.

According to a more preferred embodiment of the present invention, said microencapsulated formulation comprises or, alternatively, consists of:
- said (a.ii) microencapsulated iron comprising the iron (III) cation and said coating matrix B comprising or, alternatively, consisting of calcium alginate which microencapsulates said iron (III) cation, wherein said microencapsulated iron comprises said iron (III) cation in a % by w/w comprised in the range of from 35% to 45%, preferably 40±2%; preferably said Fe(III) cation derives from ferric saccharate; and
- said (b) cystine or a salt thereof, preferably crystalline cystine;
   and wherein said coating matrix A, which microencapsulates said (a.ii) microencapsulated iron and said (b) cystine, comprises or, alternatively, consists of sodium alginate.

According to a further preferred embodiment of the present invention, said microencapsulated formulation comprises or, alternatively, consists of:
- said (a.ii) microencapsulated iron comprising the iron (III) cation and said coating matrix B comprising or, alternatively, consisting of calcium alginate which microencapsulates said iron (III) cation, wherein said microencapsulated iron comprises said iron (III) cation in a % by w/w comprised in the range of from 35% to 45%, preferably 40±2%; preferably said Fe(III) cation derives from ferric saccharate; and
- said (b) cysteine or a salt thereof;
   and wherein said coating matrix A, which microencapsulates said (a.ii) microencapsulated iron and said (b) cysteine, comprises or, alternatively, consists of sodium alginate.

According to a further preferred embodiment of the present invention, said microencapsulated formulation comprises or, alternatively, consists of:
- said (a.ii) microencapsulated iron comprising the iron (III) cation and said coating matrix B comprising or, alternatively, consisting of calcium alginate which microencapsulates said iron (III) cation, wherein said microencapsulated iron comprises said iron (III) cation in a % by w/w comprised in the range of from 35% to 45%, preferably 40±2%; preferably said Fe(III) cation derives from ferric saccharate; and
- said (b) acetylcysteine or a salt thereof;
   and wherein said coating matrix A, which microencapsulates said (a.ii) microencapsulated iron and said (b) acetylcysteine, comprises or, alternatively, consists of sodium alginate.

According to a first alternative embodiment of the present invention, said microencapsulated formulation comprises or, alternatively, consists of:
- said (a) iron, wherein said iron is (a,ii) microencapsulated iron comprising an iron (III) or iron (II) cation and a coating matrix B comprising at least one alginate salt, preferably calcium alginate, wherein said coating matrix B microencapsulates said iron (III) or iron (II) cation;
   preferably wherein said iron (III) or iron (II) cation derives from an iron salt or complex selected from: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and preferably wherein said (a.ii) comprises Fe (III) or (II) in a % by w/w comprised in the range from 25% to 55% or from 30% to 50% or from 35% to 45% (e.g. about 40%); and
- said (b) cystine or a salt thereof or a derivative thereof, preferably crystalline cystine;
   and wherein said coating matrix A is selected from: at least one salt of an alginate (preferably sodium alginate), at least one lipid (according to any one of the embodiments described in the present invention, preferably comprising a phospholipid), and at least one polymer other than an alginate.

According to a second alternative embodiment of the present invention, said microencapsulated formulation comprises or, alternatively, consists of:
- said (a) iron, wherein said iron is (a.ii) microencapsulated iron comprising an iron (III) or iron (II) cation and a coating matrix B selected from at least one lipid (according to any one of the embodiments described in the present invention preferably comprising a phospholipid), and at least one polymer other than an alginate, wherein said coating matrix B microencapsulates said iron (III) or iron (II) cation;
   preferably wherein said iron (III) or iron (II) cation derives from an iron salt or complex selected from: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
   preferably wherein said (a.ii) comprises Fe (III) or (II) in a % by w/w comprised in the range from 25% to 55% or from 30% to 50% or from 35% to 45% (e.g. about 40%); and
- said (b) cystine or a salt thereof or a derivative thereof, preferably crystalline cystine;
   and wherein said coating matrix A is selected from: at least one salt of an alginate (preferably sodium alginate), at least one lipid (according to any one of the embodiments described in the present invention, preferably comprising a phospholipid), and at least one polymer other than an alginate.

As a process for microencapsulating the active components (a), such as (a.i) or (a.ii), and (b) with the coating matrix A comprising at least one polymer, preferably an alginate salt or at least one lipid, any process known to the man skilled in the art, preferably the microencapsulation process described hereinafter in the present invention, may be used to prepare the microencapsulated formulation of the present invention.
Advantageously, the microencapsulated formulation according to the present invention, comprising the active components (a) (such as (a.i) or (a.ii)) and (b) and a coating matrix A comprising a polymer (preferably an alginate), has a particle size (average particle diameter) comprised from 50 µm to 600 µm (for example 100 µm, 200 µm, 300 µm, 400 µm, 500 µm), preferably from 100 µm to 500 µm, more preferably from 150 µm to 400 µm.

Generally, microencapsulation is a microtechnology process by means of which individual particles of an active agent can be stored or embedded in a shell, surrounded by or coated with a continuous film made of material for producing individual particles in the range from micrometre to millimetre.
Depending on the process applied, these particles may have different sizes, shapes and specific structures. The advantage of microencapsulation lies in the fact that the core material (in the present case, the formulation comprising (a) and (b)) is completely coated and isolated from the external environment. Furthermore, microencapsulation potentially does not affect the properties of the materials of the core.

In the context of the present invention microencapsulation is preferably understood to be applied directly to particles in solid state.

Advantageously, the formulation microencapsulated with the coating matrix A comprising (a) and (b) subject of the present invention, according to any one of the embodiments (preferably (a) in the form (a.ii)), has one of the following structures (A), (B) or (A+B), as represented in figure 1 and described hereinafter:
- Core/shell structure (A) (microparticles, microcapsules): the active component or active components (pure or not) is/are confined as a core in one or more shells (single-shell or multi-shell), wherein said shell comprises or, alternatively, consists of the coating matrix.
- Structure of the matrix (B) (microbeads, microparticles): dispersion of the active component or active components (pure or not) in the microencapsulation material (or coating matrix)
- Both structures (A + B) can be combined to design the core of the matrix surrounded by a shell.

In a preferred embodiment, the microencapsulated formulation subject the present invention has said structure (A+B) (or structure of core-shell microparticles dispersed in a matrix), as represented in figure 1 and reported below: a core/shell structure (A) (microparticles, microcapsules) wherein a mixture of the active components (a) and (b) is confined as a core in one or more shells (single-shell or multi-shell), wherein said shell comprises or, alternatively, consists of the coating matrix A of the present invention: furthermore, a plurality of core/shell structures (A) are dispersed in the coating matrix A of the present invention, according to said structure (B).
Preferably, in said structure (A+B) said active component (a) is in the form (a.ii), such as iron or ferric saccharate microencapsulated with the coating matrix B.

According to a preferred example, in said structure (A+B) (or core-shell microparticles dispersed in a matrix) the microparticles with core/shell structure (A) have a mono-shell.
In a preferred embodiment, the microencapsulated formulation subject the present invention comprising (a), such as (a.i) or (a.ii), and (b) and having - as coating matrix A based on at least one alginate - preferably sodium alginate, is produced by means of a microencapsulation process (in short, microencapsulation process of the present invention) having the following steps and characteristics:
- Drying, granulation and microencapsulation combined in a single process (3 in 1) with a short drying time (for example from 5 minutes to 180 minutes, for example 10 minutes, 20 minutes, 30 minutes, 60 minutes, 90 minutes or 120 minutes), allow less physical-chemical stress for the materials to be coated.
- The process can be applied to particles of sizes up to 20 µm (for example from 0.5 µm to 20 µm, such as 1 µm, 5 µm, 10 µm, or 15 µm), as a mixture of (a) and (b) or the individual components (a) and (b).
- Multilayer coating: continuous sequence of individual spraying steps.
- The large surface of the product facilitates the application of the liquid coating or adhesive medium.
- Benefits for the environment, given that spray loss is reduced to the minimum.
- Reliable processing of even highly friable products.
- Efficient coating of complex geometries, as well as products and particles with a hollow structure.
- Even flow conditions in the container allow an even and rapid coating.
- Homogeneous particle size, shape and geometry with almost spherical granules.
- Scalable spray speed allows batches of different sizes.
- Droplet size is stable irrespective of the spray volume.

According to a preferred embodiment, the microencapsulation process of the present invention is performed by means of a fluid bed system, i.e. a microencapsulation system comprising a fluid bed process chamber (for example, a fluid bed chamber according to standard processes and machines). In said fluid-bed chamber the components to be microencapsulated (i.e. a mixture of (a) and (b), wherein preferably (a) is in form of (a.ii)), are under flow motion of a gas and, at the same time, the coating matrix A of the present invention comprising a polymer (preferably an alginate) is sprayed (emitted by a nozzle by means of spray) into said fluid-bed chamber. The result is the microencapsulation of the active components (a) and (b) in the coating matrix A, to obtain the microencapsulated formulation of the present invention. Said microencapsulation in a fluid bed is preferably performed at a process temperature comprised from 20°C to 80°C (for example 25°C, 35°C, 40°C, 50°C, 60°C, or 70°C), preferably from 30°C to 60°C.
Advantageously, said fluid-bed microencapsulation process is applied so as to provide the formulation of (a) and (b) microencapsulated in a coating matrix A (with an alginate) having said structure (A+B) of core-shell microparticles dispersed in a coating matrix (figure 1).

Forming an object of the present invention is the microencapsulated formulation of the present invention, comprising (a) iron, such as Fe(III) or Fe(ll) cation, preferably in the form of (a.i) or (a.ii) as described in the present invention, and (b) cystine or a salt or derivative thereof and having a coating matrix A comprising at least one polymer, preferably at least one alginate salt (more preferably sodium alginate) or at least one lipid, that can be obtained by means of the microencapsulation process described in the present invention.

The homogeneous coating of the microencapsulation, obtained by means of the microencapsulation process of the present invention, is suitable for protecting the active components (a) and (b) of the microencapsulated formulation of the present invention against the gastric acidic environment, and therefore increasing the intestinal absorption and blood bioavailability thereof.
The homogeneous coating of the microencapsulation, obtained by means of the processes of the present invention, is suitable for the modified release of the active components (a) and (b) of the microencapsulated formulation of the present invention: delayed release, prolonged release etc. It is also possible to confer modified release characteristics to only one or more selected active components of the formulation.
The homogeneous coating of the microencapsulation, obtained by means of the microencapsulation process of the present invention, is an effective protection against oxygen and/or moisture, which allows greater durability and also the use of sensitive active components. Furthermore, said microencapsulation isolates the particles allowing to use physically and/or chemically incompatible active components in the same product.
Lastly, the homogeneous coating of the microencapsulation, obtained by means of the process of the present invention, is suitable for masking the taste of the active components (a) and (b), in particular of iron, of the microencapsulated formulation of the present invention in a highly efficient manner which allows administration thereof even in case they have unpleasant taste and/or odour. Furthermore, possible irritant effects of said active components can be reduced.

In an embodiment of the microencapsulated formulation of the present invention, the (a):(b) weight ratio is comprised in a range from 1:99 to 20:80, preferably from 2:98 to 15:85, more preferably from 5:95 to 10:90, for example about 8:92 (equivalent to about 1:11.5),
wherein (a) is iron (III) or iron (II) cation (i.e., amount by weight of the iron cation as such), present in the formulation according to any one of the embodiments described in the present invention such as (a.i) or (a.ii), preferably (a.ii) microencapsulated iron comprising Fe(III) microencapsulated in a coating matrix B comprising calcium alginate wherein said Fe(III) is comprised at about 40% weight/weight (for example, AB-Fortis®), and wherein (b) is said cystine or salt or derivative thereof, preferably crystalline cystine, and wherein a derivative thereof is preferably selected from cysteine or acetylcysteine and salts thereof.

According to a preferred embodiment, in the microencapsulated formulation of the present invention, the (a):(b) weight ratio, as defined above, remains almost constant and preferably comprised in the range from 5:95 to 10:90 (for example about 8:92 or 1:11.5), while the by weight amount (or percentage by weight) of coating matrix A may vary depending on the final form of the microencapsulated formulation (for example, depending on the form of the composition of the invention comprising the microencapsulated formulation).

In an embodiment of the microencapsulated formulation of the present invention, the (i):(ii) weight ratio, is comprised in a range from 99:1 to 50:50, preferably from 98:2 to 70:30, more preferably from 97:3 to 85:15, for example 95±1:5±1,
wherein (i) is the formulation comprising (a) and (b),
wherein (a) is the iron (III) or iron (II) cation, or, alternatively,
wherein (a) is said (a.i) iron salt or complex comprising the iron (III) or iron (II) cation selected from the group of (a.i) as defined in the present invention, or, alternatively,
wherein (a) is said (a.ii) microencapsulated iron comprising an iron (III) or iron (II) cation microencapsulated in a coating matrix B comprising or, alternatively, consisting of at least one polymer, preferably an alginate salt, more preferably calcium alginate, or of at least one lipid; preferably wherein the iron (III) or iron (II) cation is comprised in said microencapsulated iron in a % by w/w comprised in the range from 25% to 55% or from 30% to 50% or from 35% to 45%; more preferably iron (III) microencapsulated in calcium alginate comprising iron (III) at about 40% w/w (for example, the commercial product AB-Fortis®),
and wherein (b) is cystine or a salt or derivative thereof, and wherein a derivative thereof is preferably selected from cysteine, acetylcysteine and salts thereof,
and wherein (ii) is the coating matrix A comprising at least one polymer, preferably at least one salt of an alginate (more preferably sodium alginate) or at least one lipid, wherein both (i) and (ii) are according to any one of the embodiments described in the present invention.

In a preferred embodiment of the microencapsulated formulation of the present invention, the components are comprised according to the following percentages by weight with respect to the total weight of the formulation:
- said (a) iron, such as Fe (III) or Fe (II) cation (i.e. amount by weight of iron cation as such), is comprised in a percentage comprised in the range from 1% to 9% or 15% (for example 1%, 2%, 3%, 4%, 5%, 6%, 7%, 8% or 10%), preferably from 5% to 9%, more preferably from 6% to 9% (for example about 7.5%-8%);
- said (b) cystine, preferably crystalline cystine, or a salt or derivative thereof, is comprised in a percentage comprised from 10% to 95% (for example 15%, 20%, 25%, 30%, 35%, 40%, 45%, 50%, 55%, 60%, 65%, 70%, 75%, 80%, 85% or 90%), preferably from 55% to 92%, more preferably from 70% to 90% (for example about 86%-87%); and
- said coating matrix A, comprising, or alternatively, consisting of at least one polymer, preferably a salt of an alginate, more preferably sodium alginate, or at least one lipid is comprised in a percentage comprised in the range from 1% to 8% or 15% (for example 1%, 2%, 3%, 4%, 5%, 6%, 7%, 8%, 9% or 10%), preferably from 2% to 8%, more preferably from 3% to 7% (for example about 5%-6%).

In a preferred embodiment of the microencapsulated formulation of the present invention, the components are comprised according to the following percentages by weight with respect to the total weight of the formulation:
- said (a) iron, such as (a.ii) microencapsulated iron comprising an iron (III) or (II) cation microencapsulated in said coating matrix B (preferably an iron (III) or ferric saccharate salt or complex microencapsulated in calcium alginate, comprising iron (III) at about 40% w/w; for example AB-Fortis®), is comprised in a percentage comprised in the range from 1% to 40% (for example 2%, 4%, 6%, 8%, 10%, 15%, 20%, 25%, 30%, o 35%), preferably from 15% to 20% (for example about 17%-18%);
- said (b) cystine, preferably crystalline cystine, or a salt or derivative thereof, is comprised in a percentage comprised from 10% to 95% (for example 15%, 20%, 25%, 30%, 35%, 40%, 45%, 50%, 55%, 60%, 65%, 70%, 75%, 80%, 85% or 90%), preferably from 80% to 90% (for example about 86%-87%); and
- said coating matrix A, comprising, or alternatively, consisting of at least one polymer, preferably a salt of an alginate, more preferably sodium alginate, or at least one lipid is comprised in a percentage comprised in the range from 1% to 8% or 15% (for example 1%, 2%, 3%, 4%, 5%, 6%, 7%, 8%, 9% or 10%), preferably from 3% to 7% (for example about 5%-6%).
Forming an object of the present invention is a composition (in short composition of the invention) comprising
- the microencapsulated formulation comprising (a) iron, such as (a.i) or (a.ii), and (b) cystine or a salt or derivative thereof, microencapsulated with said coating matrix A comprising at least one polymer, preferably a salt of an alginate, or at least one lipid according to any one of the embodiments described in the present invention,
- and, optionally, at least one acceptable pharmaceutical or food grade additive and/or excipient.

In a preferred embodiment of the invention, besides said microencapsulated formulation comprising (a), such as (a.i) or (a.ii), and (b) microencapsulated with said coating matrix A, comprising at least one polymer (preferably an alginate or at least one lipid), said composition subject of the invention further comprises (c) at least one vitamin B, preferably selected from (c.i) vitamin B2, (c.ii) vitamin B6, (c.iii) vitamin B9, (c.iv) vitamin B12 and mixtures thereof, and/or (d) vitamin C (or ascorbic acid or L-ascorbic acid, synonyms of vitamin C) and/or (e) vitamin E and/or (f) folic acid and/or additives/excipients.

In a preferred embodiment, said composition subject of the invention comprises
- a microencapsulated formulation comprising or, alternatively, consisting of:
   (a) iron, preferably (a.i) an iron salt or complex as defined in the present invention or, alternatively, (a.ii) iron (III) or (II) cation microencapsulated in a coating matrix B comprising an alginate salt, preferably calcium alginate, wherein the iron (III) or (II) cation is comprised in a % by w/w from 25% to 55% (for example AB-Fortis®);
   (b) cystine or a salt or derivative thereof, wherein said derivative is selected from cysteine, acetylcysteine and salts thereof, preferably crystalline cystine; and
   a coating matrix A comprising or, alternatively, consisting of at least one alginate salt, preferably selected from Na, Mg, K, ammonium and Ca alginate, more preferably sodium alginate (E401);
   and wherein said composition further comprises
- (d) at least one vitamin B selected from the group comprising or, alternatively, consisting of: (c.i) vitamin B2, (c.ii) vitamin B6, (c.iii) vitamin B9, (c.iv) vitamin B12; preferably said (d) comprises vitamin B2, vitamin B6, vitamin B9 and vitamin B12, each independently in amounts sufficient to satisfy at least or more than 100% of the NRVs (Nutrient Reference Values);
   and, optionally, at least one acceptable pharmaceutical or food grade additive and/or excipient.
In a more preferred embodiment, said composition subject of the invention comprises
- a microencapsulated formulation comprising or, alternatively, consisting of:
   (a) iron, preferably (a.i) an iron salt or complex as defined in the present invention or, alternatively, (a.ii) iron (III) or (II) cation microencapsulated in a coating matrix B comprising an alginate salt, preferably calcium alginate, wherein the iron (III) or (II) cation is comprised in a % by w/w from 25% to 55% (for example AB-Fortis®);
   (b) cystine or a salt or derivative thereof, wherein said derivative is selected from cysteine, acetylcysteine and salts thereof, preferably crystalline cystine; and
a coating matrix A comprising or, alternatively, consisting of at least one alginate salt, preferably selected from Na, Mg, K, ammonium and Ca alginate, more preferably sodium alginate (E401);
and wherein said composition further comprises:
- (c) at least one vitamin B selected from the group comprising or, alternatively, consisting of: (c.i) vitamin B2, (c.ii) vitamin B6, (c.iii) vitamin B9, (c.iv) vitamin B12; preferably said (d) comprises vitamin B2, vitamin B6, vitamin B9 and vitamin B12, each independently in amounts sufficient to satisfy at least or more than 100% of the NRVs (nutrient reference values);
- (d) vitamin C and/or (e) vitamin E;
   and, optionally, at least one acceptable pharmaceutical or food grade additive and/or excipient.

In a more preferred embodiment, said composition subject of the invention comprises
- a microencapsulated formulation comprising or, alternatively, consisting of:
   (a.ii) iron (III) cation microencapsulated in a coating matrix B comprising an alginate salt, preferably calcium alginate, wherein the iron (III) is comprised in a % by w/w from 25% to 55%, preferably from 30% to 50% or from 35% to 45% (for example AB-Fortis®);
   (b) cystine or a salt or derivative thereof, wherein said derivative is selected from cysteine, acetylcysteine and salts thereof, preferably crystalline cystine; and
   a coating matrix A comprising or, alternatively, consisting of an alginate salt, preferably sodium alginate (E401);
   and wherein said composition further comprises
- (c) vitamin B2, vitamin B6, vitamin B9 and vitamin B12, each independently in amounts sufficient to satisfy at least or more than 100% of the NRVs (for example %NRVs for each vitamin B comprised in the range from 200% to 2000%);
- (d) vitamin C in amounts sufficient to satisfy at least or more than 100% of the NRVs (for example, %NRVs comprised in the range from 300% to 800%);
- (e) vitamin E in amounts sufficient to satisfy at least or more than 100% of the NRVs (for example %NRVs comprised in the range from 300% to 800%);
   and, optionally, (f) folic acid and/or at least one acceptable pharmaceutical or food grade additive and/or excipient.

Embodiments (in short FRs) of the composition of the present invention are reported below, comprising the active components (a), such as (a.i) or (a.ii), (b), (c), such as (c.i) and/or (c.ii) and/or (c.iii) and/or (c.iv), (d) and (e), as reported in the present description:
FRa.i: (a.i), (b), (c) such as (c.i) and/or (c.ii) and/or (c.iii) and/or (c.iv);
FRa.ii: (a.ii), (b), (c) such as (c.i) and/or (c.ii) and/or (c.iii) and/or (c.iv);
FRb.i: (a.i), (b), (c) such as (c.i) and/or (c.ii) and/or (c.iii) and/or (c.iv), (d) ;
FRb.ii: (a.ii), (b), (c) such as (c.i) and/or (c.ii) and/or (c.iii) and/or (c.iv), (d);
FRc.i: (a.i), (b), (c) such as (c.i) and/or (c.ii) and/or (c.iii) and/or (c.iv), (e) ;
FRc.ii: (a.ii), (b), (c) such as (c.i) and/or (c.ii) and/or (c.iii) and/or (c.iv), (e);
FRd.i: (a.i), (b), (c) such as (c.i) and/or (c.ii) and/or (c.iii) and/or (c.iv), (d), (e) ;
FRd.ii: (a.ii), (b), (c) such as (c.i) and/or (c.ii) and/or (c.iii) and/or (c.iv), (d), (e);
FRe.i: (a.i), (b) and (d) and/or (e);
FRe.ii: (a.ii), (b) and (d) and/or (e);

Each embodiment from FRa.i to FRe.ii listed above may further comprise (f) folic acid and/or at least one acceptable pharmaceutical or food grade additive and/or excipient.

The compositions of the present invention, comprising said microencapsulated formulations (i.e. (a)+(b)+ coating matrix A) and, optionally, (c) and/or (d) and/or (e) and/or (f), may comprise at least one acceptable pharmaceutical or food grade additive and/or excipient, i.e. a substance devoid of therapeutic activity suitable for pharmaceutical or food use. In the context of the present invention, the acceptable additives and/or excipients for pharmaceutical or food use comprise all the ancillary substances known to the man skilled in the art for the preparation of compositions in solid, semi-solid or liquid form, such as, for example, diluents, solvents (including water), solubilisers, acidifiers, thickeners, sweeteners, flavour enhancers, colorants, sweeteners, lubricants, surfactants, preservatives, stabilisers, pH stabilising buffers and mixtures thereof. Preferably, said at least one acceptable pharmaceutical or food grade additive and/or excipient are selected from: isomalt, fructose, xanthan gum, glucomannan, citric acid, sucralose, acesulfame K, flavour and mixtures thereof.
In an embodiment, besides said microencapsulated formulation (i.e. (a)+(b)+ coating matrix A) and, optionally, (c) and/or (d) and/or (e) and/or (f) and/or additives, the composition of the present invention may further comprise at least one further active component selected from the group comprising or, alternatively, consisting of: anti-inflammatory agents, antioxidants, anti-radical agents, nutrients, vitamins of group D, A , prebiotics, probiotics belonging to the families of yeasts and bacteria, enzymes, immunostimulants, organic and/or inorganic salts, plant extracts.

Preferably, the microencapsulated formulations and the compositions thereof subject of the present invention are "allergen free". The term "allergen free" means that it does not have any allergen residues.

The composition subject of the present invention can be in the form of a liquid, such as solution, biphasic liquid system, dispersions or suspension of a solid in a liquid, semisolid form, such as gel, cream or foam, or in the form of a solid, such as powder, granules, microgranules, flakes, tablets or capsules.

Preferably, the compositions of the invention are formulated in a form suitable for sublingual, buccal administration through the oral (or gastroenteric) route.

In a preferred embodiment of the invention, the compositions of the invention are administered through the oral route in the form of a solid; preferably, in the form of a solid as such or in the form of a mouth-soluble (or mouth-dispersible, that dissolve in the oral cavity) solid, such as powder, granules, microgranules, flakes, tablets or chewable tablets, capsules or soft-gel, preferably tablets or granules or powders.

When the compositions of the invention are in a solid form for oral use, said solid form may be coated with gastroprotective films, for example tablets or granules.

In a further embodiment of the invention, the compositions of the invention are administered through the oral route in the form of a liquid, such as solution, biphasic liquid system, dispersions or suspension of a solid in a liquid (for example by preparing before use).

The composition of the invention or the microencapsulated formulation subject the present invention according to any one of the described embodiments, may be a pharmaceutical composition or formulation, a medical device composition or formulation, a dietary supplement, a food or novel food or nutraceutical composition invention, a composition to be added to or to be supplemented in a food or beverage.
In the context of the present invention, the expression "medical device" is used in the meaning according to the Italian Legislative Decree n° 46 dated 24 February 1997 or according to the new Medical Device Regulation (EU) 2017/745 (MDR).

Forming an object of the present invention are the microencapsulated formulations ((a)+(b)+ coating matrix A) of the present invention and the compositions of the present invention comprising said microencapsulated formulations, according to any one of the described embodiments, for use as medicament.

Forming an object of the present invention are the microencapsulated formulations comprising (a), such as (a.i) or (a.ii) and (b) and the compositions of the present invention comprising said microencapsulated formulations and, optionally, (d) and/or (e) and/or (f) and/or food or pharmaceutical grade additives/excipients, according to any one of the embodiments of the present invention, for use in a method for the preventive and/or curative treatment of anaemia or iron deficiency and diseases, disorders or symptoms related thereto, in a subject in need, wherein said treatment method provides for the administration of an effective dose of a microencapsulated formulation or relative composition subject of the present invention to said subject.

Preferably, said diseases, symptoms or disorders related to or deriving from said anaemia or iron deficiency are selected from: fatigue, weakness, asthenia, irritability, headache, insomnia, shortness of breath, breathlessness, chest pain, vertigo and dizziness, cold hands and feet, brittle nails, hair loss, accelerated heart rate, sore throat, poor appetite, tingling in legs.

Preferably, the microencapsulated formulations of the present invention and the compositions thereof are for use in the treatment of subjects with anaemia or iron deficiency such as women, pregnant or breastfeeding women, paediatric subjects, the elderly, sportsmen/sportswomen, subjects suffering from a chronic kidney disease, subjects suffering from a chronic infection, subjects suffering from arthritis and subjects suffering from tumours, subjects with high oxidative stress, smokers, diabetics, subjects suffering from hypertension, subjects undergoing haemodialysis, the elderly, dyslipidemic subjects, nephropathic subjects (dialysed and non-dialysed), subjects suffering from COPD, subjects suffering from heart failure, subjects suffering from IBD (chronic inflammatory bowel diseases), alcoholics, subjects with celiac disease, subjects suffering from metabolic syndrome.
The microencapsulated formulations of the present invention and the compositions thereof are not for use in a method for the treatment of pathological conditions related to glutathione deficiency, such as HIV (human immunodeficiency virus), COPD (chronic obstructive pulmonary disease) or cystic fibrosis.

Forming an object of the present invention are a method for the preventive and/or curative treatment of anaemia or iron deficiency and/or diseases or symptoms related thereto (as described in the present invention) in a subject in need, wherein said treatment method, provides for the administration of an therapeutically effective amount of a microencapsulated formulation or relative composition subject of the present invention to said subject.

Furthermore, forming an object of the present invention is the use (non-therapeutic) the microencapsulated compositions and formulations subject of the present invention, according to any one of the described embodiments, to increase the physical and/or mental performance of a healthy subject, such as sportsmen/sportswomen or students, for example, to increase physical resistance to physical stress, reduce time to recover forces after physical stress, and increase physical resistance to mental stress.

In the context of the present invention, the term "subject/s" is used to indicate human or animal subjects, preferably mammals (e.g. pets such as dogs, cats, horses, sheep or cattle). Preferably, the compositions of the invention are for use in treatment methods for human subjects.
Unless specified otherwise, the expression composition or formulation or other comprising a component at an amount "comprised in a range from x to y" is used to indicate that said component can be present in the composition or formulation or other at all the amounts present in said range, even though not specified, extremes of the range comprised.
Unless specified otherwise, the content of a component in a composition or formulation refers to the percentage by weight of that component with respect to the total weight of the composition or formulation. Unless specified otherwise, the indication that a composition or mixture or other "comprises" one or more components means that other components can be present besides that or those specifically indicated and the indication that a composition or mixture or other "consists" of determined components means that the presence of other components is excluded.
The expression "the therapeutically effective amount" is used to indicate an amount that can be identified by medical staff or specialised personnel for the purpose of the treatment of the subject based on objective data.

### EXAMPLES

An example of microencapsulated formulations according to the present invention are reported in Table 1 (for example, product under the trade name IRONCYS ACTIVE™ brand owned by Pharma Line, Italy).

**Table 1. (1) for example: commercial product AB-Fortis®; (2) for example: commercial product CISTINA-L BASE; (3) for example: commercial product NS ENTERIC®.**

| Microencapsulated formulation | mg per daily intake amount | % VNRs | % w/w |
|---|---|---|---|
| (a.ii) Microencapsulated iron⁽¹⁾ | 79 | 214 | 17.49 |
| (equivalent in Fe) | (30) | | |
| (b) Cystine⁽²⁾ | 350 | | 77.51 |
| Sodium alginate⁽³⁾ | 22.50 | | 5.00 |
| Total | 451.50 | | 100 |

- AB-Fortis® (registered trademark, AB-Biotics S.A., Spain) is a microencapsulated iron having the following technical characteristics: composition: 40% from iron (III) and 60% from encapsulating material (primarily calcium alginate) of which iron (III) is Fe(OH)O sucrose (ferric saccharate or ferric sucrose hydroxide complex, CAS No. 8047-67-4) and of which calcium alginate has molecular formula (C₆H₇Ca_{1/2}O₆)n and CAS No. 9005-35-0; Ingredients: alginate (from sodium alginate, E-401), calcium (from calcium acetate, E-263) 2% w/w, iron (from ferric saccharate) 40% w/w; Content: Fe (III) 38-43% dry weight ICP-OES (Inductively Coupled Plasma-optical Emission Spectroscopy), calcium (II) 2-4% dry weight (ICP-OES); Release of iron in water <1.5% (ICP-OES); Release of calcium in water <20% (ICP-OES); Moisture<5% (w/w); Particle size <20µm; Form: colloidal suspension, insoluble in water. Product as reported in patent application WO 2010/040789 A1 (incorporated in the present patent application for reference purposes) from page 6, line 26, to page 7, line 30 and Example 1; particle size according to Example 2; characteristics according to Example 3.
- CISTINA-L BASE (Polichimica, Italy) cystine CAS No. 56-89-, BRUTE FORMULA C6H12N2O4S2, MOLECULAR WEIGHT 240.30; having the following technical characteristics: Transmittance (T430) Min. 98%; pH 5.0 - 6.5; Specific rotation -215/-225°; Ammonium (NH4) Max. 0.02%; Chlorides Max. 0.020%; Sulphates (SO4) Max. 0.020%; Iron Max. 20 ppm; Heavy metals (such as Pb) Max. 10 ppm; Arsenic (As2O3) Max. 1 ppm; Loss on drying Max. 0.20%; Residue after calcination Max. 0.10%; Other amino acids Compliant; Titre 98.5 - 101.0%: Dissolved density 0.45 - 0.6 g/ml.

An example of a composition according to the present invention is reported in Table 2 (for example, product under the trade name Emacrit® Plus manufactured by Pharma Line, Italy).

**Table 2. (1) and (2) e (3): as defined in Table 1. (4): Vitamin B9 for example in the form of 5-methyltetrahydrofolate calcium salt.**

| Composition | mg per daily intake amount | % VNRs | % w/w |
|---|---|---|---|
| Components microencapsulated with sodium alginate⁽³⁾ | | | |
| (a.ii) Microencapsulated iron⁽¹⁾ | 79 | 214 | 7.71 |
| (equivalent in Fe) | (30) | | (2.93) |
| (b) Cystine⁽²⁾ | 350 | - | 34.15 |

| NON-microencapsulated components | | | |
|---|---|---|---|
| (c.i) vitamin B2 | 25 | 1785 | 2.44 |
| (c.ii) vitamin B6 | 10 | 714 | 0.97 |
| (c.iii) vitamin B9⁽⁴⁾ | 0.400 | 200 | 0.04 |
| (c.iv) vitamin B12 | 0.025 or 0.010 | 400 | 0.002 |
| (d) vitamin C | 500 | 625 | 48.78 |
| (e) vitamin E | 60 | 500 | 5.85 |
| total | 1024.425 | | |

Possible dosages of the composition of the invention according to Table 2 are as follows: one dosage unit per day, two dosage units per day taken simultaneously or one dosage unit twice a day at a distance of at least 4-6 hours, wherein said dosage unit may be a sachet comprising the composition of the invention in the form of granules or powder or, alternatively, in the form of tablet.

Examples of embodiments (in short, FRs) of the present invention are reported below:
FR1. A microencapsulated formulation with a coating matrix A,
   wherein said formulation comprises or, alternatively, consists of:
   (a) an iron, wherein said iron is an iron (III) or iron (II) cation, and
   (b) a cystine or a salt thereof or a derivative thereof, wherein said derivative is selected from cysteine, acetylcysteine and salts thereof; and
   wherein said coating matrix A comprises or, alternatively, consists of at least one alginate salt or at least one phospholipid or at least one polymer other than an alginate salt.
FR2. The formulation according to FR1, wherein said microencapsulated formulation has an average particle diameter comprised from 100 µm to 500 µm, preferably from 150 µm to 400 µm.
FR3. The formulation according to FR1 or FR2, wherein said formulation comprises or, alternatively, consists of:
   - said (a) iron, wherein said iron is an (a.i) iron salt or complex comprising an iron (III) or iron (II) cation, wherein said iron salt or complex is selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
   - said (b) cystine or a salt or a derivative thereof, wherein said derivative is selected from cysteine, acetylcysteine and salts thereof; preferably cysteine; more preferably crystalline cystine;
   and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate; more preferably sodium alginate E401.
FR4. The formulation according to any one of the FR1- FR3, wherein said formulation comprises or, alternatively, consists of:
   - said (a) iron, wherein said iron is (a.ii) microencapsulated iron comprising an iron (III) or iron (II) cation and a coating matrix B comprising at least one alginate salt, preferably calcium alginate, or at least one phospholipid or at least one polymer other than an alginate salt which microencapsulates said iron (III) or iron (II) cation;
      preferably wherein said microencapsulated iron comprises said iron (III) or iron (II) cation in a weight/weight percentage of microencapsulated iron comprised in the range from 25% to 55%, preferably from 30% to 50%, more preferably from 35% to 45%; and
   - said (b) cystine, or a salt or a derivative thereof, wherein said derivative is selected from cysteine, acetylcysteine and salts thereof; preferably cysteine; more preferably crystalline cystine;
      and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate; more preferably sodium alginate E401.
FR5. The formulation according to FR4, wherein said formulation comprises or, alternatively, consists of:
   - said (a.ii) microencapsulated iron comprising the iron (III) cation and said coating matrix B comprising or, alternatively, consisting of at least one alginate salt, preferably calcium alginate, which microencapsulates said iron (III) cation, wherein said iron (III) cation is comprised in a weight/weight percentage of microencapsulated iron comprised in the range from 25% to 55%, preferably from 30% to 50%, more preferably from 35% to 45%; and
   - said (b) cystine or a salt thereof, preferably crystalline cystine;
      and wherein said coating matrix A, which microencapsulates said (a.ii) microencapsulated iron and said (b) cystine or a salt thereof, comprises or, alternatively, consists of at least one alginate salt, preferably sodium alginate; more preferably sodium alginate E401.
FR6. The formulation according to any one of FR1- FR5, wherein an [iron (III) or (II) cation] : [cystine or a salt thereof or a derivative thereof] weight ratio is comprised in the range from 1:99 to 20:80, preferably from 2:98 to 15:85, more preferably from 5:95 to 10:90, wherein said cystine derivative is selected from cysteine, acetylcysteine and salts thereof.
FR7. A composition comprising:
   - the microencapsulated formulation according to any one of FR1- FR6;
      and, optionally,
   - at least one acceptable pharmaceutical or food grade additive and/or excipient.
FR8. The composition according to FR7, wherein besides said microencapsulated formulation according to any one of FR1- FR6, said composition further comprises at least one vitamin B; preferably selected from: vitamin B2, vitamin B6, vitamin B9, vitamin B12 and mixtures thereof.
FR9. The composition according to FR7 or FR8, wherein besides said microencapsulated formulation according to any one of FR1- FR5 and, optionally, said at least one vitamin B, said composition further comprises vitamin C and/or vitamin E.
FR10. The composition according to any one of FR7- FR9, wherein said composition comprises:
   - the microencapsulated formulation according to any one of FR1- FR6,
   - vitamin B2, vitamin B6, vitamin B9 and vitamin B12;
   - vitamin C, and
   - vitamin E.
FR11. The composition according to any one of FR6- FR9, wherein said composition is formulated for oral use; preferably for oral use in solid form, preferably in the solid form of granules or powders or tablets.
FR12. The microencapsulated formulation according to any one of FR1-FR6 or the composition according to any one of FR7- FR10 for use as medicament.
FR13. The microencapsulated formulation according to any one of FR1-FR6 or the composition according to any one of FR7- FR10 for use in a method for the preventive and/or curative treatment of anaemia or iron deficiency, and diseases, disorders or symptoms related thereto, in a subject in need;
   preferably wherein said diseases, symptoms or disorders related to or deriving from said anaemia are selected from: fatigue, weakness, asthenia, irritability, headache, insomnia, shortness of breath, breathlessness, chest pain, vertigo and dizziness, cold hands and feet, brittle nails, hair loss, accelerated heart rate, sore throat, poor appetite, tingling in legs.
FR14. Non-therapeutic use of the microencapsulated formulation according to any one of FR1- FR6 or of the composition according to any one of FR7- FR10 for increasing iron levels in a healthy subject; preferably wherein said healthy subject is a sportsman/sportswoman.
FR15. A process for the preparation of a microencapsulated formulation according to any one of FR1-FR6, wherein said process provides for the use of a fluid bed chamber.

### EXPERIMENTAL PART

### A. FIRST CLINICAL STUDY

### 1. Purpose of the clinical study

Interventional, randomised, controlled study aimed at investigating the efficacy of the product according to Table 2 (composition according to the present invention, commercial product Emacrit® plus) in patients recruited for first-stage prosthetic hip and knee surgery to improve the preoperative iron profile. The study is conducted according to Good Clinical Practice recommendations.

### 2. Clinical study design

The population consists of 82 subjects recruited for first hip or knee prosthetic surgery, randomised to two groups: surgery and control. The duration of the study is set at 2 years, including the preparation of the study for the Ethics Committee, recruitment, and results.

While the patients in the control group follow the clinical routine, the experimental arm with the product according to Table 2 provides for supplementation with the product for about two months prior to elective surgery, with baseline blood assessment (T0) of complete blood count, serum iron, transferrin levels, ferritinemia, TIBC.

At the end of the treatment period, after about two months, blood samples (T1) are collected to evaluate any changes in the iron profile prior to the indicated surgery.

In postoperative stage, recruited subjects are monitored (T2 and others) for biochemical, functional, clinical parameters.

### 3. End-points of the clinical study

The primary outcome of this clinical trial is the evaluation of the preoperative variation (from T0 to T1) in haemoglobin between the two groups after iron treatment through the oral route with the product according to Table 2.

For example, an average increase in haemoglobin in the treated group - higher by about 0.80 g/dl with respect to the one observed in the untreated group, with a standard deviation of 1.2 g/dl - can be considered clinically significant. Furthermore, we assume an alpha error of 0.05 (two-tailed) and a statistical test power of 80%. Based on these values, a sample size of 37 subjects per group is obtained, which - increased by 10% due to losses at follow-up - brings the sample size to 41 subjects per group.

### B. SECOND CLINICAL STUDY

### 1. Purpose of the clinical study

Interventional, randomised, controlled study aimed at investigating the efficacy of the product according to Table 2 (composition according to the present invention) in elderly patients suffering from sideropenic anaemia. The study is conducted according to Good Clinical Practice recommendations.

Iron deficiency is considered the primary cause of nutritional anaemia, insufficient levels of vitamins B12, C, E, folates and riboflavin are associated with this deficiency. One third of elderly subjects suffer from forms of anaemia that cannot be defined clearly. Oxidative stress, which affects the survival of erythrocytes and iron metabolism, may play a significant role.

### 2. Objectives of the clinical study

### 2.1. Primary objectives

- Increase in the level of haemoglobin
- Enhancement in iron profile

### 2.2. Secondary objectives

- Confirmation of product tolerability
- Enhancement of the quality of life
- Appreciation of the therapy

### 3. Study design

- Patients to be enrolled: at least 20 patients
- Inclusion criteria:
   - Male and female subjects
   - Above 80 years of age
   - Haemoglobin < 12 g/dL
   - Serum iron< 65 (µg/dL
   - Ferritin < 50 (µg/L
- Exclusion criteria:
   - Known allergy to one or more ingredients of Emacrit® plus
   - Ferritin > 600 ng/mL
   - Active infections
   - Malignant blood diseases
   - Patients on dialysis or estimated glomerular filtration rate < 30mL/min/1.73m2
   - Other therapies for anaemia or blood transfusions in the two weeks prior to starting taking Emacrit® plus.

### 4. Materials and methods

### 4.1. Treatment

One sachet per day of the compound according to the present invention (compound according to Table 2, commercial product Emacrit® plus) dissolved in a glass of water away from meals.

### 4.2. Execution:

- of the complete blood count (Haematocrit, Haemoglobin, Red blood cells, Mean corpuscular volume of red blood cells, Mean red blood cell haemoglobin content (MCH), Mean red blood cell haemoglobin concentration (MCHC), Red blood cell volume distribution (RDW), White blood cells, Platelets);
- of the dosage of: either iron, or ferritin, or transferrin, or percent transferrin saturation, or vitamin B12, or folate; if possible also: either vitamin B2, or vitamin B6;
- Evaluation of the quality of life.
- Evaluation of tolerability and adherence to treatment.

All evaluations are carried out at recruitment (prior to initiation of therapy T0), after one month of treatment with Emacrit® plus (T1), and after three months of treatment with Emacrit® plus (T2).

## Claims

1. A microencapsulated formulation with a coating matrix A,
wherein said formulation comprises or, alternatively, consists of:
(a) an iron, wherein said iron is an iron (III) or iron (II) cation, and
(b) a cystine or a salt thereof or a derivative thereof, wherein said derivative is selected from cysteine, acetylcysteine and salts thereof; and
wherein said coating matrix A comprises or, alternatively, consists of at least one alginate salt or at least one phospholipid or at least one polymer other than an alginate salt.

2. The formulation according to claim 1, wherein said microencapsulated formulation has an average particle diameter comprised from 100 µm to 500 µm, preferably from 150 µm to 400 µm.

3. The formulation according to claim 1 or 2, wherein said formulation comprises or, alternatively, consists of:
- said (a) iron, wherein said iron is an (a.i) iron salt or complex comprising an iron (III) or iron (II) cation, wherein said iron salt or complex is selected from the group comprising or, alternatively, consisting of: iron pyrophosphate or ferric diphosphate, ferrous sulphate, ferrous gluconate, ferrous lactate, ferric ammonium citrate, ferrous fumarate, ferrous succinate and ferric saccharate; preferably ferric saccharate; and
- said (b) cystine or a salt or a derivative thereof, wherein said derivative is selected from cysteine, acetylcysteine and salts thereof; preferably cysteine; more preferably crystalline cystine;
and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate; more preferably sodium alginate E401.

4. The formulation according to any one of claims 1-3, wherein said formulation comprises or, alternatively, consists of:
- said (a) iron, wherein said iron is (a.ii) microencapsulated iron comprising an iron (III) or iron (II) cation and a coating matrix B comprising at least one alginate salt, preferably calcium alginate, or at least one phospholipid or at least one polymer other than an alginate salt which microencapsulates said iron (III) or iron (II) cation;
preferably wherein said microencapsulated iron comprises said iron (III) or iron (II) cation in a weight/weight percentage of microencapsulated iron comprised in the range from 25% to 55%, preferably from 30% to 50%, more preferably from 35% to 45%; and
- said (b) cystine, or a salt or a derivative thereof, wherein said derivative is selected from cysteine, acetylcysteine and salts thereof; preferably cysteine; more preferably crystalline cystine;
and wherein said coating matrix A comprises or, alternatively, consists of at least one alginate salt selected from the group comprising or, alternatively, consisting of: sodium alginate, potassium alginate, magnesium alginate, ammonium alginate, calcium alginate and mixtures thereof; preferably sodium alginate; more preferably sodium alginate E401.

5. The formulation according to claim 4, wherein said formulation comprises or, alternatively, consists of:
- said (a.ii) microencapsulated iron comprising the iron (III) cation and said coating matrix B comprising or, alternatively, consisting of at least one alginate salt, preferably calcium alginate, which microencapsulates said iron (III) cation, wherein said iron (III) cation is comprised in a weight/weight percentage of microencapsulated iron comprised in the range from 25% to 55%, preferably from 30% to 50%, more preferably from 35% to 45%; and
- said (b) cystine or a salt thereof, preferably crystalline cystine;
and wherein said coating matrix A, which microencapsulates said (a.ii) microencapsulated iron and said (b) cystine or a salt thereof, comprises or, alternatively, consists of at least one alginate salt, preferably sodium alginate; more preferably sodium alginate E401.

6. The formulation according to any one of claims 1-5, wherein an [iron (III) or (II) cation] : [cystine or a salt thereof or a derivative thereof] weight ratio is comprised in the range from 1:99 to 20:80, preferably from 2:98 to 15:85, more preferably from 5:95 to 10:90, wherein said cystine derivative is selected from cysteine, acetylcysteine and salts thereof.

7. A composition comprising:
- the microencapsulated formulation according to any one of claims 1- 6;
and, optionally,
- at least one acceptable pharmaceutical or food grade additive and/or excipient.

8. The composition according to claim 7, wherein besides said microencapsulated formulation according to any one of claims 1-6, said composition further comprises at least one vitamin B; preferably selected from: vitamin B2, vitamin B6, vitamin B9, vitamin B12 and mixtures thereof.

9. The composition according to claim 7 or 8, wherein besides said microencapsulated formulation according to any one of claims 1-5 and, optionally, said at least one vitamin B, said composition further comprises vitamin C and/or vitamin E.

10. The composition according to any one of claims 7-9, wherein said composition comprises:
- the microencapsulated formulation according to any one of claims 1-6,
- vitamin B2, vitamin B6, vitamin B9 and vitamin B12;
- vitamin C, and
- vitamin E.

11. The composition according to any one of claims 6-9, wherein said composition is formulated for oral use; preferably for oral use in solid form, preferably in the solid form of granules or powders or tablets.

12. The microencapsulated formulation according to any one of claims 1-6 or the composition according to any one of claims 7-10 for use as medicament.

13. The microencapsulated formulation according to any one of claims 1-6 or the composition according to any one of claims 7-10 for use in a method for the preventive and/or curative treatment of anaemia or iron deficiency, and diseases, disorders or symptoms related thereto, in a subject in need;
preferably wherein said diseases, symptoms or disorders related to or deriving from said anaemia are selected from: fatigue, weakness, asthenia, irritability, headache, insomnia, shortness of breath, breathlessness, chest pain, vertigo and dizziness, cold hands and feet, brittle nails, hair loss, accelerated heart rate, sore throat, poor appetite, tingling in legs.

14. Non-therapeutic use of the microencapsulated formulation according to any one of claims 1-6 or of the composition according to any one of claims 7-10 for increasing iron levels in a healthy subject; preferably wherein said healthy subject is a sportsman/sportswoman.

15. A process for the preparation of a microencapsulated formulation according to any one of claims 1-6, wherein said process provides for the use of a fluid bed chamber.
